(19) Europäisches Patentamt
European Patent Office
Office européen des brevets

(11) Veröffentlichungsnummer: **0 092 677**
**B1**

(12) **EUROPÄISCHE PATENTSCHRIFT**

(45) Veröffentlichungstag der Patentschrift: **19.06.85**

(21) Anmeldenummer: **83102828.7**

(22) Anmeldetag: **22.03.83**

(51) Int. Cl.⁴: **C 07 C 45/36**, C 07 C 47/575, C 07 C 47/542, B 01 J 23/68, B 01 J 23/89, B 01 J 23/88, B 01 J 27/18

(54) **Verfahren zur Herstellung von substituierten Benzaldehyden.**

(30) Priorität: **28.04.82 CH 2597/82**
**24.02.83 CH 1029/83**

(43) Veröffentlichungstag der Anmeldung:
**02.11.83 Patentblatt 83/44**

(45) Bekanntmachung des Hinweises auf die Patenterteilung:
**19.06.85 Patentblatt 85/25**

(84) Benannte Vertragsstaaten:
**AT BE CH DE FR GB IT LI NL**

(56) Entgegenhaltungen:
**EP - A - 0 009 239**
**GB - A - 1 110 616**
**GB - A - 1 152 215**
**US - A - 4 293 444**
**US - A - 4 309 355**

**CHEMICAL ABSTRACTS, Band 87, 1977, Seite 656, Nr. 134285b, Columbus, Ohio, USA**
**V.K. Sharma & S.K. Bhattacharya "Indian Journal of Technology Vol. 14, Oct. 1976 pp 488-491**

(73) Patentinhaber: **F. HOFFMANN-LA ROCHE & CO. Aktiengesellschaft, CH-4002 Basel (CH)**

(72) Erfinder: **Nösberger, Paul, Dr., Passwangstrasse 1, CH-4127 Birsfelden (CH)**

(74) Vertreter: **Urech, Peter, Dr. et al, Grenzacherstrasse 124 Postfach 3255, CH-4002 Basel (CH)**

**EP 0 092 677 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung von substituierten Benzaldehyden, und zwar von Benzaldehyden der Formel

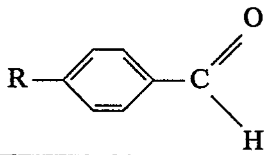 (I)

worin R eine Methoxy- oder eine tert. Butyl-Gruppe darstellt.

Die Verbindungen der Formel I sind bekannte Stoffe, insbesondere mit Zwischenproduktcharakter.

Das Verfahren ist dadurch gekennzeichnet, daß man eine Verbindung der Formel

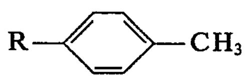 (II)

worin R obige Bedeutung besitzt,
in Anwesenheit von Sauerstoff oder Sauerstoff enthaltenden Gasen und eines Metalloxyd-Katalysators der Zusammensetzung

$$MoMe^1_aMe^2_bO_x \qquad III$$

worin Me[1] für Kupfer oder Silber und Me[2] für eines oder mehrere der Elemente Ti, Zr, Fe, Co, Ni, Zn, Sn, Pb, Sb, Bi, B, P oder eine seltenes Edelmetall steht, a 0,2 bis 1,0, b 0 bis 0,5 und x die Anzahl der zum Absättigen der Valenzen der vorhandenen anderen Elemente erforderlichen Sauerstoffatome bezeichnet,
bei Temperaturen von 350° bis 600° C oxidiert.

Das Verfahren ist durch hohe Selektivität

$$\left(\text{Anteil der Verbindung I im Reaktionsprodukt, also } \frac{\text{gebildete Mole I}}{\text{umgesetzte Mole II}} \times 100\right)$$

auch noch bei hohem Umsatz (Verhältnis umgesetztes Ausgangsmaterial II/eingesetztes Ausgangsmaterial II ist hoch) gekennzeichnet.

$Me^1$ ist vorzugsweise Kupfer. $Me^2$ ist vorzugsweise Eisen, Cer, Zink oder Zirkon und besonders bevorzugt Zinn. Der Begriff »seltenes Erdmetall« soll im übrigen die Metalle der Ordnungszahlen 21, 39, 57—71 bezeichnen.

Das molare Verhältnis von Mo zur Summe der übrigen Metalle ist vorzugsweise 1—3, insbesondere 1,05—1,4.

Das molare Verhältnis von $Me^1$ zu $Me^2$ ist vorzugsweise größer als ca. 2, insbesondere beträgt es 5—30.

Zum Erzielen besonders ausgeprägter Aktivitäten des Katalysators hat sich — insbesondere im Falle von b=0 oder von geringeren Werten von b — Zugabe von Talk oder Siliciumdioxid mit großer spezifischer Oberfläche, z. B. >100 $m^2/g$, als günstig erwiesen.

Der Katalysator kann in reiner Form vorliegen, mit einem internen Trägermaterial gemischt sein oder auf einem inerten, geformten Trägermaterial (vorzugsweise in Form von Kugeln) fixiert sein. Beispiele inerter Trägermaterialien sind $\alpha$-Aluminiumoxid, Keramik, Kieselguhr, Diatomeenerde, Glas, Siliciumcarbid, ausgeglühtes Siliciumdioxid und dergleichen. Vorzugsweise werden Katalysatoren verwendet, die auf einem inerten Trägermaterial fixiert sind. Bevorzugte Trägermaterialien sind $\alpha$-Aluminiumoxid und Keramik.

Das erfindungsgemäße Verfahren kann in der Wirbelschicht oder vorzugsweise in einem Festbettreaktor durchgeführt werden.

Als Oxidationsmittel wird erfindungsgemäß Sauerstoff oder, vorzugsweise, ein sauerstoffhaltiges Gas verwendet.

Der Ausdruck »sauerstoffhaltiges Gas« bedeutet hierbei allgemein ein Gemisch von Sauerstoff und einem inerten Gas, wie beispielsweise Stickstoff, Kohlendioxid, Argon und Wasserdampf. Der Sauerstoffgehalt im Eduktstrom ist nicht kritisch. Im allgemeinen beträgt er jedoch etwa 1—40 Vol.% und vorzugsweise etwa 5—15 Vol.%. Besonders bevorzugt ist die Verwendung von Luft, der gewünschtenfalls zur Mäßigung des Reaktionsverlaufs und zum Abführen von Reaktionswärme Stickstoff und/oder Abgase aus dem Reaktor (nach Abtrennung des Aldehyds) zugesetzt werden können.

Die Menge Sauerstoff oder sauerstoffhaltiges Gas ist nicht kritisch. Um eine ausreichende Umsetzung zu erreichen, sollte dem Reaktor pro Mol Edukt II mindestens etwa 1 Mol Sauerstoff zugeführt

werden. Mit Vorteil wird jedoch mit einem Überschuß an Sauerstoff gearbeitet, beispielsweise mit etwa 200—2000% und vorzugsweise mit etwa 300—1000% Überschuß.

Der Gehalt des Toluols II im Ausgangsgemisch kann innerhalb eines breiten Bereichs variieren, wobei natürlich die Selbstentzündungstemperatur und die Explosionsgrenze von II beachtet werden müssen. Im allgemeinen soll die Eduktkonzentration aber etwa 0,5—10, vorzugsweise 1—3 Vol% betragen.

Im allgemeinen sollen die Kontaktzeiten im Bereich von 0,1 bis 10 Sekunden liegen, wobei der Bereich von etwa 0,1 bis 0,5 Sekunden bevorzugt ist.

Der Druck ist nicht besonders kritisch. Vorteilhafterweise wird die Reaktion bei Atmosphärendruck durchgeführt, doch kommen auch höhere oder tiefere Drucke in Frage.

Die erfindungsgemäße Oxydation des Edukts II ist stark exotherm und ist auch temperaturabhängig. Die optimale Temperatur ist abhängig vom verwendeten Katalysator, der Sauerstoff- und Eduktkonzentration im Eduktstrom, der Gasgeschwindigkeit, der Form und Größe des Reaktors und dergleichen. Im allgemeinen wird in einem Temperaturbereich von etwa 350 bis etwa 600° C, vorzugsweise etwa 400 bis etwa 500° C gearbeitet. Besonders bevorzugt ist ein Temperaturbereich von etwa 430 bis etwa 470° C.

Als Reaktormaterialien kommen grundsätzlich alle üblicherweise verwendeten Werkstoffe in Frage, welche Edukt und Produkt unter den Reaktionsbedingungen praktisch nicht angreifen, wie beispielsweise rostfreier Stahl, Glas, Keramik und dergleichen.

Die Katalysatormenge ist nicht kritisch. Die eingesetzte Menge ist insbesondere abhängig von der Temperatur, der Form und Größe des Reaktors und der eingesetzten Menge Edukt. Die Optimierung wird zweckmäßig so durchgeführt, daß der Katalysator und gewünschtenfalls inertes Trägermaterial in den Reaktor (vorzugsweise einen Festbettreaktor) eingeführt werden und dann die Verweilzeit und die Temperatur so eingestellt werden, daß der Umsatz an Edukt optimal ist. Falls der Katalysator auf einen geformten Träger (z. B. Keramikkugeln) aufgebracht wird, so beträgt die Katalysatormenge etwa 2—10%, vorzugsweise 4—8% der eingesetzten Trägermenge.

Zwecks Gewinnung des Produkts I wird der Produktstrom gekühlt und es werden die kondensierbaren Anteile auf diese Weise abgetrennt. Ein Teil der Abgase kann, wie oben erwähnt, wieder dem Reaktor als Verdünnungsmittel zugeführt werden. Die kondensierten organischen Produkte werden auf übliche Weise, z. B. destillativ aufgetrennt und das nicht umgesetzte Ausgangsmaterial zusammen mit den tiefsiedenden Nebenprodukten rezyklisiert.

Die Katalysatoren können leicht auf an sich bekannte Weise hergestellt werden, also z. B. durch Zusammengeben einer wäßrigen Lösung von $Me^1/Me^2$-Salzen (vorzugsweise der Nitrate) mit einer wäßrigen Lösung von Ammoniumheptamolybdat/Ammoniak, Filtration, Waschen mit Wasser, gegebenenfalls Fixieren auf einem Träger und Calcinieren, z. B. bei 450—600° C. Auf die Filtration und das Waschen kann aber auch verzichtet werden. In diesem Fall wird die Calcinierung am besten im Reaktor und einem ausreichenden Gasstrom (z. B. Luft) durchgeführt. Dabei wird die Temperatur zunächst auf 200° C und hierauf langsam (entsprechend der Entwicklung nitroser Gase) auf die gewünschte Calcinierungstemperatur erhöht. Das Calcinieren bedingt, daß die Metalle üblicherweise in der höchsten Oxydationsstufe vorliegen.

In der Formel III ist x deshalb auch die Zahl der Sauerstoffatome, die sich formal aus der Anzahl der vorhandenen Metall-, Bor- und Phosphoratome und deren höchsten Oxydationsstufe ergibt.

## Beispiel 1

Der Reaktor besteht aus einem elektrisch beheizten Verdampferrohr (400° C, Länge 20 cm) und einem Reaktionsrohr (Länge 60 cm, Durchmesser 3,5 cm), das von einem Luftbad mit Umwälzung umgeben ist und am oberen Ende mit dem Verdampferrohr verbunden ist. Die Rohrteile sind aus rostfreiem Stahl gefertigt. Das Verdampferrohr, sowie der obere und untere Teil des Reaktionsrohres werden mit Keramikkugeln, die mittleren 34 cm des Reaktionsrohres mit Katalysator gefüllt. Dieser wird folgendermaßen hergestellt: In einem Dragierkessel von 1,5 l Inhalt werden 600 g Keramikkugeln mit einem Durchmesser von 4—5 mm mit einer Aufschlämmung von 0,25 g Talk in 20 ml destilliertem Wasser belegt. Auf den vorbehandelten Träger werden nun 21,0 g Ammoniumheptamolybdat-tetrahydrat, 24,6 g Kupfer-II-nitrattrihydrat, 1,6 g Zink-II-chlorid und 3,7 g Cer-III-nitrathexahydrat in 550 ml destilliertem Wasser dragiert. Drehzahl der Pfanne, Luftstrom (400° C) und Dosierung der Metallsalzlösung werden so eingestellt, daß sich im Träger eine Temperatur von 60—70° C einstellt.

330 ml dieses Katalysators werden in den Reaktor eingefüllt und bei einem Luftstrom von 220 l/h auf 200° C geheizt, bis die Bildung nitroser Gase abklingt. Anschließend wird die Temperatur allmählich auf 500° C erhöht und der Katalysator während drei Stunden bei dieser Temperatur calciniert; formale Zusammensetzung des Katalysators:

$MoCu_{0,86}Zn_{0,10}Ce_{0,07}O_x$.

Die Luftbadtemperatur wird auf 390° C erniedrigt und dem Verdampfer stündlich 24 ml p-tert.-Butyl-

toluol und 220 l Luft zugeführt. Die Analyse der Reaktionsprodukte ergibt einen Umsatz von 58% und eine Selektivität bzgl. p-tert.-Butyl-benzaldehyd von 35%. Die Ausbeute an Aldehyd

$$\left(\frac{\text{gebildeter Aldehyd I}}{\text{eingesetztes II}} \times 100 \left[\frac{\text{Mol}}{\text{Mol}}\right]\right)$$

beträgt 20%.

## Beispiel 2

Wie Beispiel 1, jedoch wird die Luftzufuhr auf 120 l/h reduziert und es werden 100 l/h Abgase zurückgeführt. Vom zurückgeführten p-tert.-Butyl-toluol werden 31% umgesetzt. Die Ausbeute an Aldehyd beträgt 13%, die Selektivität ist 43%.

## Beispiel 3

Ein Katalysator mit der formalen Zusammensetzung

$MoCu_{0,45}Zr_{0,05}O_x$

wird folgendermaßen hergestellt:

Zu einer gerührten Lösung von 32,1 g Ammoniumheptamolybdat-tetrahydrat und 8 g Ammoniak (25%) in 150 ml destilliertem Wasser gibt man eine Lösung von 19,8 g Kupfer-II-nitrat-trihydrat und 3,9 g Zirkonium-IV-nitratpentahydrat in 35 ml destilliertem Wasser. Der Niederschlag wird abgenutscht, mit 60 ml destilliertem Wasser gewaschen, aufgeschlämmt in 600 ml destilliertem Wasser und analog Beispiel 1 auf 600 g Keramikkugeln aufgebracht. Anschließend wird der Katalysator 16 Stunden bei 550° C calciniert.

Die Prüfung der katalytischen Wirksamkeit erfolgt analog Beispiel 1. Bei einer Luftbadtemperatur von 410°C werden dem Reaktor stündlich 200 l Luft und 24 ml p-tert.-Butyl-toluol zugeführt. Davon werden 58% umgesetzt. Die Ausbeute an Aldehyd beträgt 22%, die Selektivität ist 38%.

## Beispiel 4

Wie Beispiel 3, jedoch wird die Luftbadtemperatur auf 420° C erhöht und die Luftzufuhr auf 100 l/h reduziert. Zusätzlich werden 100 l/h Abgase aus dem Reaktor zurückgeführt. Der Umsatz beträgt 50%, Ausbeute und Selektivität bzgl. Aldehyd 21 bzw. 42%.

## Beispiel 5

Ein Katalysator mit der formalen Zusammensetzung

$MoCu_{0,45}Zn_{0,05}O_x$

wird hergestellt durch Zugabe einer Lösung von 14,5 g Kupfer-II-nitrat-trihydrat und 0,9 g Zink-II-chlorid in 26 ml destilliertem Wasser zu einer gerührten Lösung von 23,6 g Ammoniumheptamolybdat-tetrahydrat/5,9 g Ammoniak (25%) in 110 ml destilliertem Wasser. Der Niederschlag wird abgenutscht, mit ca. 40 ml destilliertem Wasser gewaschen, aufgeschlämmt in 400 ml destilliertem Wasser, analog Beispiel 1 auf 440 g Keramikkugeln aufgebracht und anschließend 16 Stunden bei 550° C calciniert.

Die Prüfung der katalytischen Wirksamkeit erfolgt analog Beispiel 1. Die Luftbadtemperatur wird auf 440° C eingestellt. Dem Reaktor werden stündlich 50 l Luft, 150 l Stickstoff und 24 ml p-tert.-Butyl-toluol zugeführt. Davon werden 29% umgesetzt. Die Selektivität bzgl. p-tert.-Butyl-benzaldehyd beträgt 49%, die Ausbeute 14%.

## Beispiel 6

Ein Katalysator der formalen Zusammensetzung

$MoCu_{0,43}Fe_{0,036}Ce_{0,036}O_x$

wird hergestellt durch Zugabe einer Lösung von 18,8 g Kupfer-II-nitrat-trihydrat, 2,6 g Eisen-III-nitratnonahydrat und 2,8 g Cer-III-nitrat-hexahydrat in 35 ml destilliertem Wasser zu einer gerührten Lösung von 32,1 g Ammoniumheptamolybdat-tetrahydrat in 150 ml destilliertem Wasser. Anschließend werden 8 g Ammoniak (25%) zugegeben, der Niederschlag abgenutscht, mit ca. 60 ml destilliertem Wasser gewaschen, aufgeschlämmt in 600 ml destilliertem Wasser, analog Beispiel 1 auf 600 g Keramikkugeln fixiert und 16 Stunden bei 550° C calciniert.

Die Prüfung der katalytischen Wirksamkeit erfolgt analog Beispiel 1. Bei einer Luftbadtemperatur von 415°C, einer Luft- und Stickstoffzufuhr von je 100 l/h werden dem Reaktor stündlich 24 ml p-tert.-Butyl-toluol zugeführt. Davon werden 57% umgesetzt. Die Selektivität bzgl. p-tert.-Butyl-benzaldehyd beträgt 39%, die Ausbeute 22%.

## Beispiel 7

Ein Katalysator mit der formalen Zusammensetzung

$MoCu_{0,43}O_x$

wird hergestellt durch Zugabe einer Lösung von 29 g Kupfer-II-nitrat-trihydrat in 40 ml destilliertem Wasser zu einer gerührten Lösung von 49,4 g Ammoniumheptamolybdat-tetrahydrat und 10 g Ammoniak (25%) in 190 ml destilliertem Wasser. Der Niederschlag wird abgenutscht, mit ca. 40 ml destilliertem Wasser gewaschen, aufgeschlämmt in 600 ml destilliertem Wasser, analog Beispiel 1 auf 600 g Keramikkugeln aufgebracht und 16 Stunden bei 500° C calciniert.

Die Prüfung der katalytischen Wirksamkeit erfolgt analog Beispiel 1. Die Luftbadtemperatur wird auf 430° C eingestellt. Stündlich werden dem Reaktor 400 l Luft und 36 ml p-tert.-Butyl-toluol zugeführt. Davon werden 31% umgesetzt. Die Selektivität bzgl. p-tert.-Butyl-benzaldehyd beträgt 52%, die Ausbeute ist 16%.

## Beispiel 8

Ein Katalysator mit der formalen Zusammensetzung

$MoCu_{0,9}O_x$

wird hergestellt durch Zugabe einer Lösung von 27,3 g Kupfer-II-nitrat-trihydrat in 50 ml destilliertem Wasser zu einer gerührten Lösung von 22,24 g Ammoniumheptamolybdat-tetrahydrat in 350 ml destilliertem Wasser. Die entstandene Aufschlämmung wird analog Beispiel 1 auf 600 g Keramikkugeln aufgebracht. Der Katalysator wird in ein Rohr (Ø 5 cm) eingefüllt und in einem Luftstrom von 200 l/h bei 200° C gehalten bis die Nebelbildung im Abgas abklingt (ca. 1h). Anschließend wird die Temperatur allmählich auf 400° C erhöht. Vor Gebrauch wird der Katalysator 16 Stunden bei 550° C calciniert.

Die katalytische Wirksamkeit wird wie folgt ermittelt:

Ein elektrisch heizbares vertikales Rohr aus rostfreiem Stahl (Ø 2,4 cm, Länge 60 cm) wird in der Mitte mit 50 ml Katalysator und an den beiden Enden mit Keramikkugeln (Ø 6 mm) gefüllt. Nun wird der Katalysator auf 420° C aufgeheizt und dem Reaktor stündlich 100 l Luft und 12 ml p-tert.-Butyl-toluol zugeführt. Die Heizung des Rohres wird so eingestellt, daß das Temperaturmaximum in der Reaktionszone 450° C beträgt. Die Analyse der Reaktionsprodukte ergibt einen Umsatz von 13,2%, eine Selektivität bzgl. p-tert.-Butyl-benzaldehyd von 66,9% und eine Ausbeute von 8,8%.

## Beispiel 9

Der Katalysator wird wie in Beispiel 8 beschrieben hergestellt und geprüft, jedoch werden zur Ammoniumheptamolybdat-tetrahydrat-Lösung noch 6 g Talk gegeben. Vom eingesetzten p-tert.-Butyl-toluol werden 25,8% umgesetzt. Die Selektivität bzgl. p-tert.-Butyl-benzaldehyd beträgt 55,1%, die Ausbeute 14,2%.

## Beispiel 10

Ein Katalysator mit der formalen Zusammensetzung

$MoCu_{0,85}Sn_{0,05}O_x$

wird hergestellt durch Zugabe einer Lösung von 25,97 g Kupfer-II-nitrat-trihydrat in 50 ml destilliertem Wasser und einer Lösung von 1,24 g Zinn-II-chlorid-dihydrat in 20 ml Alkohol zu einer gerührten Lösung

von 22,24 g Ammoniumheptamolybdat-tetrahydrat in 300 ml destilliertem Wasser. Im übrigen erfolgen Herstellung und Prüfung gemäß Beispiel 8. Vom eingesetzten p-tert.-Butyl-toluol werden 30,4% umgesetzt. Die Selektivität bzgl. p-tert.-Butyl-benzaldehyd beträgt 59,5%, die Ausbeute 18,1%.

Beispiel 11

Die Prüfung des Katalysators erfolgt gemäß Beispiel 10, jedoch werden dem Reaktor stündlich bloß 6 ml p-tert.-Butyl-toluol zugeführt. Davon werden 51,2% umgesetzt. Die Selektivität bzgl. p-tert.-Butylbenzyldehyd beträgt 48,3% die Ausbeute 24,7%.

Beispiel 12

Der Katalysator wird gemäß Beispiel 10 hergestellt und geprüft, jedoch das Temperaturmaximum in der Reaktionszone nun 490°C. Vom eingesetzten p-tert.-Butyl-toluol werden 42,4% umgesetzt. Die Selektivität bzgl. p-tert.-Butyl-benzaldehyd beträgt 68%, die Ausbeute 28,8%.

**Patentansprüche**

1. Verfahren zur Herstellung von Benzaldehyden der Formel

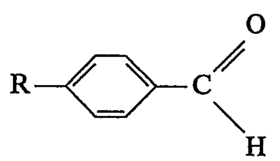

(I)

worin R Methoxy- oder tert.-Butyl darstellt,
dadurch gekennzeichnet, daß man eine Verbindung der Formel

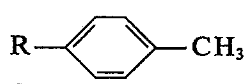

(II)

worin R obige Bedeutung besitzt,
in Anwesenheit von Sauerstoff oder Sauerstoff enthaltenden Gasen und eines Metalloxyd-Katalysators der Zusammensetzung

$$MoMe^1_aMe^2_bO_x \qquad III$$

worin $Me^1$ für Kupfer oder Silber und $Me^2$ für eines oder mehrere der Elemente Ti, Zr, Fe, Co, Ni, Zn, Sn, Pb, Sb, Bi, B, P oder ein seltenes Erdmetall steht, a 0,2 bis 1,0, b 0 bis 0,5 und c die Anzahl der zum Absättigen der Valenzen der vorhandenen anderen Elemente erforderlichen Sauerstoffatome bezeichnet,
bei Temperaturen von 350° bis 600° C oxidiert.

2. Verfahren nach Anspruch 1, dadurch gekennzeichnet, daß $Me^2$ in der Formel III für eines oder mehrere der Elemente Ti, Zr, Fe, Co, Ni, Zn, Sn, Sb, Bi, B, P oder ein seltenes Erdmetall steht.

3. Verfahren nach Anspruch 1 oder 2, dadurch gekennzeichnet, daß der Katalysator zusätzlich Talk, oder Siliciumdioxid mit großer spezifischer Oberfläche enthält.

4. Verfahren nach einem der Ansprüche 1 bis 3, dadurch gekennzeichnet, daß $Me^1$ Kupfer ist.

5. Verfahren nach einem der Ansprüche 1 bis 4, dadurch gekennzeichnet, daß $Me^2$ Eisen, Cer, Zink oder Zirkon ist.

6. Verfahren nach einem der Ansprüche 1 bis 4, dadurch gekennzeichnet, daß $Me^2$ Zinn ist.

7. Verfahren nach einem der Ansprüche 1 bis 6, dadurch gekennzeichnet, daß R tert.-Butyl darstellt.

8. Verfahren nach einem der Ansprüche 1 bis 7, dadurch gekennzeichnet, daß man die Reaktion bei einer Temperatur von etwa 400° C bis etwa 500° C durchführt.

9. Verfahren nach einem der Ansprüche 1 bis 8, dadurch gekennzeichnet, daß man die Reaktion bei einer Temperatur von etwa 430° C bis etwa 470° C durchführt.

10. Verfahren nach einem der Ansprüche 1 bis 9, dadurch gekennzeichnet, daß die Reaktion bei Atmosphärendruck durchgeführt wird.

11. Verfahren nach einem der Ansprüche 1 bis 10, dadurch gekennzeichnet, daß man einen Katalysator verwendet, welcher auf einem inerten Trägermaterial, vorzugsweise auf $\alpha$-Aluminiumoxid oder Keramik, fixiert ist.

12. Verfahren nach einem der Ansprüche 1 bis 11, dadurch gekennzeichnet, daß die Kontaktzeit

ungefähr 0,1 bis ungefähr 10 Sekunden beträgt.

13. Verfahren nach einem der Ansprüche 1 bis 12, dadurch gekennzeichnet, daß man die Reaktion mit Luft durchführt, welcher gewünschtenfalls noch Stickstoff und/oder Abgase aus dem Reaktor zugesetzt werden.

**Claims**

A process for the manufacture of benzaldehydes of the formula

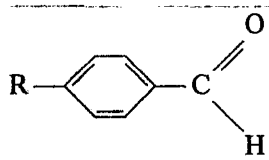

(I)

wherein R represents methoxy or tert.-butyl,
characterized by oxidizing a compound of the formula

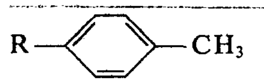

(II)

wherein R has the above significance,
in the presence of oxygen or oxygen-containing gases and a metal oxide catalyst of the composition

$$MoMe^1_aMe^2_bO_x \qquad III$$

wherein $Me^1$ stands for copper or silver and $Me^2$ stands for one or more of the elements Ti, Zr, Fe, Co, Ni, Zn, Sn, Pb, Sb, Bi, B, P or a rare earth metal, a denotes 0,2 to 1,0, b denotes 0 to 0,5 and x denotes the number of oxygen atoms require to satisfy the valencies of the other elements present,
at temperatures of 350° to 600° C.

2. A process according to claim 1, characterized in that $Me^2$ in formula III stands for one or more of the elements Ti, Zr, Fe, Co, Ni, Zn, Sn, Sb, Bi, B, P or a rare earth metal.

3. A process according to claim 1 or 2, characterized in that the catalyst additionally contains talc, or silicon dioxide with a large specific surface area.

4. A process according to any one of claims 1 to 3, characterized in that $Me^1$ is copper.

5. A process according to any one of claims 1 to 4, characterized in that $Me^2$ is iron, cerium, zinc or zirconium.

6. A process according to any one of claims 1 to 4, characterized in that $Me^2$ is tin.

7. A process according to any one of claims 1 to 6, characterized in that R represents tert.-butyl.

8. A process according to any one of claims 1 to 7, characterized in that the reaction is carried out at a temperature of about 400° C to about 500° C.

9. A process according to any one of claims 1 to 8, characterized in that the reaction is carried out at a temperature of about 430° C to about 470° C.

10. A process according to any one of claims 1 to 9, characterized in that the reaction is carried out at atmospheric pressure.

11. A process according to any one of claims 1 to 10, characterized in that there is used a catalyst which is fixed to an inert carrier material, preferably to $\alpha$-aluminium oxide or ceramics.

12. A process according to any one of claims 1 to 11, characterized in that the contact time amounts to approximately 0,1 to approximately 10 seconds.

13. A process according to any one of claims 1 to 12, characterized in that the reaction is carried out with air to which are added, if desired, nitrogen and/or exhaust gases from the reactor.

**Revendications**

1. Procédé de préparation de benzaldéhydes de formule

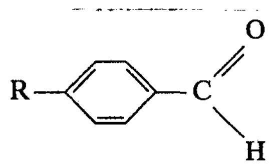

(I)

dans laquelle R repésente un groupe méthoxy ou tert-butyle,

caractérisé en ce que l'on oxyde à des températures de 350 à 600° C un composé de formule

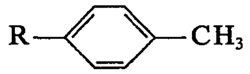 (II)

dans laquelle R a la signification indiquée ci-dessus, en présence d'oxygène ou de gaz contenant de l'oxygène et d'un catalyseur à base d'oxydes métalliques, de composition

$MoMe^1_aMe^2_bO_x$ III

dans laquelle $Me^1$ représente le cuivre ou l'argent et $Me^2$ représente un ou plusieurs des éléments Ti, Zr, Fe, Co, Ni, Zn, Sn, Pb, Sb, Bi, B, P ou un métal des terres rares, a a une valeur de 0,2 à 1,0, b une valeur de 0 à 0,5 et x est le nombre des atomes d'oxygène nécessaires pour saturer les valences des autres éléments présents.

2. Procédé selon la revendication 1, caractérisé en ce que, dans la formule III, $Me^2$ représente un ou plusieurs des éléments Ti, Zr, Fe, Co, Ni, Zn, Sn, Sb, Bi, B, P ou un métal des terres rares.

3. Procédé selon la revendication 1 ou 2, caractérisé en ce que le catalyseur contient en outre du talc ou de la silice à grande surface spécifique.

4. Procédé selon l'une des revendications 1 à 3, caractérisé en ce que $Me^1$ représente le cuivre.

5. Procédé selon l'une des revendications 1 à 4, caractérisé en ce que $Me^2$ représente le fer, le cérium, le zinc ou le zirconium.

6. Procédé selon l'une des revendications 1 à 4, caractérisé en ce que $Me^2$ représente l'étain.

7. Procédé selon l'une des revendications 1 à 6, caractérisé en ce que R représente un groupe tert-butyle.

8. Procédé selon l'une des revendications 1 à 7, caractérisé en ce que l'on effectue la réaction à une température d'environ 400° C à environ 500° C.

9. Procédé selon l'une des revendications 1 à 8, caractérisé en ce que l'on effectue la réaction à une température d'environ 430° C à environ 470° C.

10. Procédé selon l'une des revendications 1 à 9, caractérisé en ce que l'on effectue la réaction à pression atmosphérique.

11. Procédé selon l'une des revendications 1 à 10, caractérisé en ce que l'on utilise un catalyseur fixé sur une matière de support inerte, de préférence de l'alpha-alumine ou une matière céramique.

12. Procédé selon l'une des revendications 1 à 11, caractérisé en ce que la durée de contact est d'environ 0,1 à environ 10 seconds.

13. Procédé selon l'une des revendications 1 à 12, caractérisé en ce que l'on effectue la réaction à l'aide d'air auquel on peut encore ajouter, si on le désire, de l'azote et/ou les gaz résiduaires sortant du réacteur.